# DEMANDE DE BREVET EUROPEEN

(11) **EP 4 269 555 A1**
(43) Date de publication de la demande: **01.11.2023**
(21) Numéro de dépôt: 22170591.6
(22) Date de dépôt: 28.04.2022
(51) Int. Cl.: C12M 1/26, C12M 1/12

(54) **DISPOSITIF DE PRÉLÈVEMENT ASEPTIQUE**

(71) Demandeur: BECARV, 6730 Tintigny (BE)
(72) Inventeur: Papleux, Michel, 1473 Glabais (BE); Pepinster, Etienne, 5024 Gelbressée (BE)
(74) Mandataire: Calysta NV

(57) **Abrégé**

L'invention se rapporte à un dispositif de prélèvement aseptique comprenant:
- Un moyen de prélèvement (1) étant agencé pour prélever de manière aseptique une matière première (2) qui est contenue dans un premier contenant (3) d'un volume prédéterminé, et
- Une chambre de réception (4) qui présente un espace confinable (5) et étant agencée pour permettre une décontamination de celle-ci par le passage d'au moins un fluide dans ledit espace confinable (5), ladite chambre de réception (4) étant munie d'une ouverture obturable (6), d'un moyen d'accueil (7) orientable dans l'espace confinable (5).

## Description

La présente invention se rapporte à un dispositif de prélèvement aseptique agencé pour permettre une prolifération d'une matière première (dans un milieu de culture) ou pour permettre une mise en culture cellulaire ou microbienne.

Généralement, les dispositifs de prélèvement sont utilisés dans l'industrie pharmaceutique ou dans le domaine des biotechnologies pour prélever et transférer une matière première qui peut proliférer dans un milieu de culture. Cette matière première est souvent en solution. Celle-ci est ainsi prélevée d'un contenant, par exemple au moyen d'une seringue ou d'une pipette, et éventuellement transférée vers un autre contenant afin de permettre une prolifération dans un milieu de culture.

Ainsi, la matière première peut être prélevée d'un contenant puis transférée vers un autre, en fonction du but visé dans le domaine technique considéré. Dans certains domaines d'application comme ceux évoqués ci-avant, il est nécessaire que la matière première puisse être prélevée d'un contenant de manière aseptique et éventuellement transférée de manière aseptique vers un autre contenant, notamment lorsqu'une mise en culture (cellulaire ou batérienne) est prévue. Ces moyens de prélèvement représentent une des clefs de voute de ces industries pharmaceutiques et biotechnologiques étant donné qu'ils font partie de la première étape du processus. Cette étape étant capitale puisqu'elle nécessite de pouvoir prélever et transférer une matière première de manière aseptique, ce qui conditionne le reste du procédé.

Plus précisément, le prélèvement aseptique d'une matière première est capital dans différents domaines d'application, en particulier dès qu'une matière première est prédisposée à pouvoir proliférer dans un milieu de culture. Ceci est communément appelé « reproduction/amplification cellulaire » ou « culture bactérienne ». Parmi ces mises en culture, l'homme du métier connait aussi la mise en culture d'agents infectieux.

Un autre exemple se rapporte aux cellules souches mésenchymateuses humaines (hMSC) qui sont des celllules multipotentes pouvant également proliférer (comme les cellules indifférenciées). Celles-ci peuvent se différencier en différents types de cellules comme les cellules de la moelle, du cartilage, du tendon, du muscle ou encore les cellules neuronales. Les cellules hMSC présentent de nombreux bénéfices thérapeutiques et font l'objet de plusieurs recherches, comme la thérapie cellulaire ou la régénération de tissus.

Un autre exemple où la reproduction d'une matière première peut être judicieuse concerne le domaine de l'immunothérapie cellulaire dont le traitement consiste à produire des cellules CAR-T (en anglais : « Chimeric Antigenic Receptor - T») qui vise à combattre le cancer en s'appuyant sur le propre système immunitaire du patient. Une fois produites, les cellules CAR-T sont multipliées dans le dessein d'être administrées au patient.

En outre, une mise en culture peut permettre une production de vaccins ou encore une application dans le domaine de la thérapie génique.

De nos jours, la reproduction cellulaire ou bactérienne ou la culture d'agents infectieux constitue un atout majeur dans le développement du secteur des biotechnologies pour n'en citer que quelques exemples.

Typiquement, une mise en culture de cellules ou d'agents infectieux peut être réalisée en prélevant la matière première dans des petits contenants de préservation. Ces contenants de préservations peuvent être des ampoules ou des cryotubes pour cellules, qui sont généralement conservés à très basse température (entre -70 °C et -196 °C). Ce processus nommé la cryopréservation permet de suspendre la plupart des activités biologiques et des réactions chimiques et ainsi d'en conserver le contenu. Il est évident qu'en cas de dilution de la matière première ou de formulation, une température comprise entre -4°C et la température ambiante sera suffisante.

Les cellules ou agents infectieux sont généralement transférés vers des contenants plus grands, où sont présentes les conditions environnementales propices à leur croissance et reproduction. Ces contenants de mise en culture peuvent être des boîtes de pétri, des flasques contenant du milieu de culture ou encore des bioréacteurs contenant un milieu de culture. Cela permet ainsi à partir d'un faible volume d'obtenir une grande quantité de cellules ou d'agents infectieux afin de permettre la production de molécules, de médicaments, de vaccins, d'organismes génétiquement modifiés ou la mise en place de thérapie génique afin de traiter certaines maladies génétiques.

On peut imaginer que le protocole de prélèvement et la mise en culture de cellules ou d'agents infectieux dans l'industrie pharmaceutique ou dans le domaine des biotechnologies soit relativement exigeant étant donné que l'étape de prélèvement constitue la toute première étape pour permettre une mise en culture efficace et excempt de germes, bactéries non désirés dans le milieu de culture.

Pourtant, actuellement, la matière première est extraite manuellement avec une seringue dans un caisson sous flux laminaires. L'utilisateur désinfecte avec une lingette imbibée d'alcool par exemple le contenant de préservation (flacon). Ensuite, celui-ci peut ouvrir le contenant en verre, par exemple en brisant la tête de celui-ci, pour prélever manuellement au moyen d'une seringue la suspension qui est ensuite transférée vers un autre contenant, par exemple pour une mise en culture.

Avec ce type de manipulation, il ne peut être exclu que l'utilisateur contamine l'environnement autour de la solution à prélever, en particulier lorsque celle-ci est en contact avec cet environnement. L'utilisation du flux laminaire ne peut totalement exclure l'absence de contamination particulière et biologique à cause du design de celui-ci.

Malheureusement, chacune des étapes expliquées ci-avant (décontamination, ouverture, prélèvement et transfère) est propice à l'introduction de contaminants. Ainsi, une surface mal décontaminée, une erreur humaine de manipulation ou une défaillance dans le dispositif de flux laminaires entrainent des contaminations. Ces dernières ont pour conséquence d'entrainer des retards et des surcoûts importants, la culture devenant alors inexploitale. Sur un marché, souvent à flux tendu, il est de plus en plus difficile d'assurer que toutes ces étapes soient réalisées de manière aseptique. Aussi, l'utilisation de flux laminaire dans un caisson adapté est couteux pour l'utilisateur et ne permet pas de garantir un prélèvement aseptique de manière sûr et systématique dans le cadre d'une production industrielle.

De plus, ces méthodes peuvent présenter des risques importants pour le manipulateur qui peut s'exposer à des agents infectieux hautement contagieux et pathogènes lors des différentes manipulations.

Pour ces raisons, il existe un réel besoin de pouvoir assurer un prélèvement aseptique qui soit fiable, efficace, simple et rapide, tout en diminuant les risques d'échanges de contaminants de l'utilisateur vers la solution / suspension à prélever ou inversement.

Il existe donc un réel besoin de fournir un dispositif qui permet de prélever une matière première d'un premier contenant (de préservation) aisément, efficacement et de manière fiable, tout en limitant au maximum les risques de contaminations telles qu'évoquées ci-avant.

Pour résoudre ce problème, la présente invention fournit un dispositif de prélèvement aseptique pour une prolifération d'une matière première (2) (dans un milieu de culture) ou pour une mise en culture cellulaire ou microbienne, le dispositif comprenant :
- Un moyen de prélèvement (1) d'une matière première(2), de préférence en solution, agencée pour proliférer dans un milieu de culture, ledit moyen de prélèvement (1) étant agencé pour prélever de manière aseptique la matière première (2) qui est contenue dans un premier contenant (3) d'un volume prédéterminé, et
- Une chambre de réception (4) qui présente un espace confinable (5) et qui est agencée pour permettre une décontamination de celle-ci par le passage d'au moins un fluide dans ledit espace confinable (5), ladite chambre de réception (4) étant munie :
   ∘ d'une ouverture obturable (6) agencée pour un passage dudit premier contenant (3) dans ledit espace confinable (5), et lorsqu'elle est obturée, est agencée pour mettre en confinement la chambre de réception (4),
   ∘ d'un moyen d'accueil (7) orientable dans l'espace confinable (5) et étant agencé pour réceptionner ledit premier contenant (3) et le positionner en direction dudit moyen de prélèvement (1), ce dernier étant agencé pour prélever de manière aseptique ladite solution (2) contenue dans ledit premier contenant (3) et agencé pour la transférer de manière aseptique vers un deuxième contenant (8) présentant un volume supérieur au volume dudit premier contenant (3).

Le dispositif selon la présente invention permet de prélever une matière première (2), de préférence en solution, issue d'un premier contenant (3) (préférentiellement de préservation) d'un volume prédéterminé, de manière aseptique. Cela permet de minimiser au maximum les risques de contaminations à la fois de la matière première (2) (en solution) à prélever et de l'utilisateur du dispositif, avec des rendements de productions attractifs.

Le dispositif a pour avantage de pouvoir être un dispositif à usage unique, simple d'utilisation et moins couteux que les solutions actuelles, en particulier celles nécessitant l'utilisation de caisson sous flux laminaires. Le dispositif selon l'invention est portatif, ce qui facilite son implémentation sur site (industriel).

Aussi, le dispositif selon l'invention est configuré de façon à pouvoir être sélectivement confinable.

En effet, la chambre de réception (4) peut être confinée en fermant l'ouverture obturable (6), par un élément de fermeture, par exemple à l'aide d'un bouchon, d'une membrane, d'un septum, d'une vanne, etc. Ceci permet de choisir à quel moment le dispositif doit être confiné ou non en fonction de l'étape de manipulation.

Lorsque la chambre de réception (4) présente un espace confiné (5), celle-ci est également en milieu aseptique.

Selon le dispositif de la présente invention, le moyen de prélèvement (1) peut se situer soit dans l'espace confinable (5) et donc dans la chambre de réception (4), soit en dehors de la chambre de réception (4).

Dans le premier cas de figure, l'espace confinable (5) s'étend jusqu'au moyen de prélèvement (1). Ainsi, la chambre de réception (4) et le moyen de prélèvement (1) peuvent alors être confinés en fermant l'ouverture obturable (6), par un élément de fermeture, par exemple à l'aide d'un bouchon, d'une membrane, d'un septum, d'une vanne. Ceci permet de choisir à quel moment le dispositif doit être confiné ou non en fonction de l'étape de manipulation. Le moyen de prélèvement (1) et la chambre de réception (4) peuvent ainsi être confinés et en milieu aseptique.

De manière alternative, le dispositif selon la présente invention couvre aussi la possibilité d'avoir le moyen de prélèvement (1) qui se situe à côté (adjacent) de l'espace confinable (5), c'est-à-dire en dehors de la chambre de réception (4). Le moyen de prélèvement (1) forme alors une première partie confinée du dispositif de la présente invention et la chambre de réception (4) forme la deuxième partie confinable du dispositif selon l'invention. Le moyen de prélèvement (1) étant préférentiellement directement (agencé pour être) relié (en communication fluidique) à la chambre de réception (4) ou indirectement relié à celle-ci au moyen d'un élément intermédiaire de connexion (fluidique). Le dispositif selon l'invention pouvant comprendre plusieurs parties.

Cette option alternative fournie par le dispositif selon l'invention permet aussi de sélectivement confiner certaines parties du dispositif. Ainsi, le moyen de prélèvement (1) se situe dans un espace confiné et étanche (la première partie), adjacent à celui de la chambre de réception (4) qui est située dans la deuxième partie confinable du dispositif. Dans ce mode de réalisation, il est alors possible de confiner ou non la chambre de réception (4), tout en maintenant le moyen de prélèvement (1) dans un espace confiné et étanche tout au long de la manipulation et en fonction des besoins. De préférence, une communication fluidique est donc possible entre la chambre de réception (4) et le moyen de prélèvement (1) selon ce mode alternatif.

Ainsi, la chambre de réception (4) peut être confinée en fermant l'ouverture obturable (6), par un élément de fermeture, par exemple à l'aide d'un bouchon, d'une membrane, d'un septum, d'une (électro)vanne, etc.

Ces modes de réalisations combinables entre eux offre une flexibilité à l'utilisateur, ce qui est particulièrement avantageux.

La chambre de réception du dispositif selon l'invention peut également être décontaminée à tout moment de manière aisée et efficace.

Ainsi, le dispositif selon la présente invention permet de disposer d'un système fermé (confiné) qui permet à la fois de prélever et de transférer de manière aseptique une matière première (2) qui peut proliférer dans un milieu de culture.

Au vu de ce qui précède, le dispositif de prélèvement de la présente invention fournit une solution efficace (rapide), simple et fiable pour prélever (et éventuellement transférer) de manière aseptique une matière première (2) qui est agencée pour proliférer dans un milieu de culture.

Dans un mode de réalisation préféré selon la présente invention, ledit moyen de prélèvement (1) est adjacent audit espace confinable (5) de ladite chambre de réception (4) avant prélèvement, et est de préférence dans un milieu confiné et étanche. Ce mode permet de faciliter le prélèvement de la matière première (2) et assurer de manière efficace un prélèvement aseptique. Le moyen de prélèvement (1) est préférentiellement situé en dehors de la chambre de réception (4) avant prélèvement. Le moyen de prélèvement (1) forme alors avantageusement une première partie confinée du dispositif de la présente invention et la chambre de réception (4) forme la deuxième partie confinable du dispositif selon l'invention. Le moyen de prélèvement (1) étant préférentiellement directement (agencé pour être) relié à la chambre de réception (4) ou (agencé pour) être indirectement relié à celle-ci au moyen d'un élément intermédiaire de connexion (fluidique). Le dispositif selon l'invention pouvant comprendre plusieurs parties.

Cette option alternative fournie par le dispositif selon l'invention permet de plus sélectivement confiner certaines parties du dispositif. Ainsi, le moyen de prélèvement (1) se situe dans un espace confiné et étanche (la première partie) par rapport à la chambre de réception (4) et adjacent à celle-ci (deuxième partie du dispositif). Dans ce mode de réalisation, il est alors possible de confiner ou non la chambre de réception (4), tout en maintenant le moyen de prélèvement (1) dans un espace confiné et étanche tout au long de la manipulation et en fonction des besoins. De préférence, une communication fluidique est établie entre la chambre de réception (4) et le moyen de prélèvement (1) selon ce mode avantageux.

Ainsi, le moyen de prélèvement (1) peut être confiné indépendamment de l'espace confinable (5) de la chambre de réception (4). Cela permet de diminuer les risques éventuels de contaminations et de permettre un contrôle minutieux du moyen de prélèvement (1) et de l'espace confinable (5) de la chambre de réception (4).

Aussi, le moyen de prélèvement (1) se trouve dans un milieu aseptique.

Ainsi, la chambre de réception (4) peut être confinée en fermant l'ouverture obturable (6), par un élément de fermeture, par exemple à l'aide d'un bouchon, d'une membrane, d'un septum, d'une (électro)vanne, etc.

Cette flexibilité offerte à l'utilisateur est particulièrement avantageuse.

Dans un mode de réalisation préféré selon la présente invention, ledit moyen de prélèvement (1) est séparé de la chambre de réception (4), de préférence par un élément refermable (9) choisi dans le groupe comprenant un septum, une membrane et tout autre élément équivalent qui permet une ouverture et fermeture de manière étanche, tout en assurant un prélèvement aseptique. Une (électro)vanne pourrait également être utilisée dans ces conditions préférées.

Un élément refermable (9) permet d'isoler la chambre de réception (4) du moyen de prélèvement (1) et facilite en même temps l'accès à l'espace confinable (5). Cela permet d'encore diminuer les risques de contaminations. Il est aussi possible d'effectuer plusieurs rinçages et/ou décontaminations de l'espace (5) lorsque celui-ci est confiné. Une communication fluidique entre le moyen de prélèvement (1) et la chambre de réception (4) est préférentiellement établie afin de faciliter le prélèvement de la matière première (2).

Les paragraphes précédents et descriptif y afférent se rapportant au mode de réalisation dans lequel le moyen de prélèvement (1) est adjacent à la chambre de réception (4) sont également applicables ici.

De préférence, lors dudit prélèvement, une partie dudit moyen de prélèvement (1) se situe dans l'espace confinable (5) de la chambre de réception (4). Cela permet de prélever la matière première (2) du premier contenant (3), tout en limitant le temps et le degré d'exposition du moyen de prélèvement (1) dans l'espace confinable (5). Plus précisément, ce mode de réalisation est effectué lorsque l'espace (5) de la chambre de réception (4) est confiné et étanche par rapport au milieu environnant extérieur.

Il est préférable d'éviter toute introduction du moyen de prélèvement (1) dans l'espace confinable (5) de la chambre de réception lorsque celle-ci est ouverte au milieu environnant.

Plus préférentiellement, le moyen de prélèvement (1) se situe dans une chambre de prélèvement (11) qui présente un moyen de guidage (12) dudit moyen de prélèvement (1). Cela permet de guider plus facilement le moyen de prélèvement (1) afin de faciliter la manipulation lors du prélèvement.

De manière avantageuse, ledit moyen de guidage (12) est agencé pour déplacer ledit moyen de prélèvement (1), de préférence, selon un axe médian de ladite chambre de prélèvement (11) en direction de ladite chambre de réception (4). Cela permet d'encore plus faciliter le guidage du dispositif de prélèvement (1) dans une direction de prélèvement prédéterminée.

Selon un mode particulièrement préféré, la chambre de prélèvement (11) présente un corps flexible (11a) qui présente une position de repos et une position de prélèvement. Ainsi, lorsque le corps flexible (11a) est en position de repos, tout le moyen de prélèvement (1) se situe dans la chambre de prélèvement (11). Lorsque le corps flexible (11a) est en position de prélèvement, le moyen de prélèvement (1) est déplacé dans l'espace confiné (5) de la chambre de réception (4) pour être en contact avec la matière première (2) à prélever (en solution). Quand le prélèvement est réalisé, le corps flexible (11a) est agencé pour passer à nouveau en position de repos.

Par un système de pompage ou d'aspiration ou de piston ou tout autre système de prélèvement qui garantit un prélèvement aseptique, la matière première (2) peut de préférence être prélevée et transférée vers le deuxième contenant (8). Ce dernier peut comprendre un milieu de culture qui va être mélangé avec la matière première (2) de façon à permettre une amplification de la matière première (2).

De manière alternative, ce corps flexible (11a) peut aussi être un piston qui permet aussi de fournir un environnement confiné dans lequel se situe le moyen de prélèvement. Le fonctionnement indiqué ci-avant est alors similaire s'agissant des positions de repos et de prélèvement.

Avantageusement, le moyen de guidage (12) présente une gorge (12a) qui s'étend longitudinalement sur une surface extérieure (12b) dudit moyen de guidage (12a) pour faire coulisser ledit moyen de prélèvement (1) pour qu'une des extrémités (1a) dudit moyen de prélèvement (1) soit introduite dans ladite chambre de réception (4).

De manière encore plus avantageuse, ledit moyen de prélèvement (1) présente un corps (13), de préférence un corps de pompe (13), agencé pour permettre le passage de ladite matière première (2) et qui est muni à l'une de ses extrémités d'une aiguille (14) ou d'un conduit. La présence d'une aiguille (14) ou d'un conduit permet au moyen de prélèvement (1) d'être facilement inséré à l'intérieur du premier contenant (3), et constitue donc un moyen qui permet de laisser passer un fluide par exemple par aspiration. L'aiguille sera préférée pour son efficacité car elle assure avec simplicité un prélèvement aseptique.

De manière préférée, ainsi, l'aiguile (14) peut être remplacée par un conduit qui permettrait un prélèvement et transfert efficace. En effet, l'aiguille ou le conduit a un rôle plutôt passif dans le dispositif étant donné qu'elle ou il n'assure pas le retrait de la matière première (2) mais permet plutôt un accès aisé et efficace jusqu'au fond du premier contenant (3) (flacon). Tout autre moyen équivalent conviendrait également.

Notons que l'aiguille sera préférée pour son efficacité.

Le dispositif selon l'invention permet un prélèvement de manière aseptique dans un système fermé / confiné (fournit par).

Alternativement, le moyen de prélèvement (1) est situé dans un environnement confiné et étanche, et la chambre de réception (4) dans un espace confinable (5) distinct.

Dans un mode de réalisation préféré selon la présente invention, ladite chambre de prélèvement (11) est confinée et étanche. Ce mode de réalisation permet de conférer au moyen de prélèvement (1) un environnement isolé de celui de l'espace confinable (5) de la chambre de réception (4). Ainsi, lorsque la chambre de réception (4) est ouverte, la chambre de prélèvement (11) (et le moyen de prélèvement (1)) reste confinée et étanche, ce qui procure une liberté d'action lors des manipulations du dispositif, tout en évitant toute contamination du moyen de prélèvement (1).

De plus et de manière préférentielle, la chambre de réception (4) présente au moins une partie de sa surface en matière flexible (15), ladite partie étant éventuellement adjacente audit moyen d'accueil (7), qui est agencé pour traverser ladite au moins une partie (15) de la surface de la chambre de réception (4) en matière flexible. La partie flexible (15) permet de faire en sorte que le moyen d'accueil (7) soit orientable suite à des manipulations aisées et, de préférence dans tout l'espace confinable (5). Cette partie en matière flexible (15) permet avantageusement un mouvement rotationnel, de pivotement ou/et un mouvement longitudinal du moyen d'accueil (7). Cela signifie qu'il n'est pas exclu que la chambre de réception (4) présente une partie de sa surface en matière rigide qui soit légèrement flexible par rapport à la partie en matière flexible. Il est préféré d'avoir une chambre de réception (4) qui présente une partie de sa surface qui soit déformable dans l'espace (partie en matière flexible) et une autre partie qui le soit moins (partie en matière rigide) afin de pouvoir offrir un certain maintien d'une partie des éléments de la chambre de réception (4).

Selon un mode avantageux, le dispositif selon l'invention comprend un deuxième contenant (8) adjacent à ladite chambre de réception (4) et qui est reliée par ledit moyen de prélèvement (1) à ladite chambre de réception (4). Ainsi, le deuxième contenant (8) est en communication fluidique avec le moyen de prélèvement (1), ce qui permet de transférer la matière première (2) vers ce deuxième contenant (8) qui contient un milieu de culture propice à une reproduction cellulaire ou à tout autre type d'amplification d'une matière première (2).

De manière avantageuse, une connexion (communication fluidique) peut être établie entre le deuxième contenant (8) et le moyen de prélèvement (1) et la chambre de réception (4) de manière sélective pour minimiser la présence de contaminants lors des manipulations.

Préférentiellement, ledit deuxième contenant (8) présente un volume supérieur à celui du premier contenant (3), ledit deuxième contenant (8) étant agencé pour permettre une mise en culture (amplification d'une matière première(2)), lorsque ladite solution prélevée (2) dudit premier contenant (3) est transférée dans ledit deuxième contenant (8). Cela permet à partir d'un petit contenant (3) d'ensemencer un plus grand contenant (8), pour permettre une amplification de la matière première (2), en particulier une reproduction cellulaire.

Dans un mode de réalisation préféré selon la présente invention, le moyen d'accueil (7) présente une première partie (7a) qui s'étend dans ledit espace confinable (5) et une deuxième partie (7b) qui s'étend à l'extérieur de ladite chambre de réception (4) sous la forme d'un moyen de guidage (10), de préférence selon un axe médian qui traverse ladite chambre de réception (4). Cela permet à l'utilisateur de facilement guider manuellement ou automatiquement le moyen d'accueuil en orientant cette deuxième partie qui se présente sous la forme d'un moyen de guidage (10) à l'extérieur de la chambre de réception (4), sans risque de contamination.

De manière encore plus préférée, la partie sous forme de moyen de guidage (10) est en communication fluidique avec ladite chambre de réception (4). Cela permet de relier un moyen d'évacuation (18) de fluide, par exemple en reliant un conduit à l'une des extrémités de cette deuxième partie sous forme de moyen de guidage (10). L'accès à la chambre de réception (4) est ainsi facilité. Cet accès étant localisé selon un axe médian de la chambre de réception (4), de préférence à l'opposé de l'ouverture obturable (6).

La chambre de réception (4) est avantageusement agencée pour être (ou est) en communication fluidique avec au moins un des éléments suivants ou pris en combinaison:
- Un moyen d'alimentation d'au moins un fluide de décontamination (16)
- Un moyen d'alimentation d'au moins un fluide de rinçage (17),
- Un moyen d'alimentation d'au moins un fluide de séchage (19),
- Un premier moyen d'évacuation d'au moins un fluide (18), ledit moyen d'évacuation (18) étant éventuellement directement relié à l'une des extrémités (7c) dudit moyen d'accueil (7),
- Et un deuxième moyen d'évacuation de fluide (de séchage) (20).

De cette façon, la chambre de réception (4) peut être préparée avant de réceptionner le premier contenant (3) afin d'assurer que l'espace confinable (5) puisse permettre d'assurer un prélèvement aseptique lorsque l'ouverture (6) est refermée après introduction du premier contenant (3) dans l'espace confinable.

Ce mode préféré implique que la chambre de réception (4) peut être en communication fluidique avec au moins un des éléments précités (16, 17, 18, 19, 20).

De préférence, la chambre de réception (4) comprend un moyen d'ouverture (22) du premier contenant (3), le moyen d'ouverture est de préférence situé dans l'espace confinable (5). Le moyen d'ouverture (22) est agencé pour permettre un accès à la matière première (2) du premier contenant (3). Le moyen d'ouverture (22) peut par exemple briser une des extrémités du premier contenant (3) (contenant pouvant être en verre) ou déverrouiller / dévisser à l'aise de stries un bouchon, ou encore décapsuler le premier contenant (3). Cette option additionnelle liée à la présence dudit moyen d'ouverture (22) permet ainsi d'accéder à la matière première (2) contenue dans tout type de contenant (3), en particulier lorsque le premier contenant (3) est logé dans la chambre de réception (4).

De manière particulièrement préférée, ledit moyen d'accueil (7) est moulé en une seule pièce. Aussi, le moyen d'accueil (7) peut être usiné à partir d'un bloc de matière plastique ou métallique.

Le dispositif selon l'invention peut par exemple être fabriqué par un procédé à impression 3D.

Dans un mode de réalisation préféré selon la présente invention, le dispositif est un dispositif à usage unique, de préférence portatif.

Dans un mode de réalisation préféré selon la présente invention, le premier contenant (3) présente un volume inférieur à 20 ml, de préférence inférieur à 15 ml ou supérieur à 2 ml. De manière préférée, un volume compris entre 2 et 20 ml.

Le premier contenant (3) est de préférence choisi dans le groupe constitué d'une ampoule, d'un cryotube, d'un flacon (pouvant provenir de la société Aseptic Technologies). La matière du premier contenant (3) peut être faite en verre, en plastique.

De manière encore un plus avantageuse, le dispositif selon l'invention peut fonctionner à l'aide d'un système automatisé, voire robotisé.

D'autres formes de réalisation du dispositif selon l'invention sont indiquées dans les revendications annexées.

Le terme « **aseptique** » se rapporte aux conditions dans lesquelles sont effectuées des actions de prélèvement et de transfert. Dans le cadre de la présente invention, il est proposé un dispositif de prélèvement qui permet de prélever une matière première de manière aseptique. Autrement dit, chaque action qui peut être réalisée à l'aide du dispositif selon l'invention se fait de manière aseptique, ce qui permet de maintenir le milieu (par exemple l'espace confiné) aseptique. Ceci s'applique aussi au moyen de prélèvement qui est agencé pour prélever et transférer de manière aspetique la matière première.

Dans le cadre de la présente invention, l'expression « **matière première** » englobe le terme souche ou toute autre matière biologique qui peut proliférer dans un milieu de culture. La « matière première » est de préférence une solution, une suspension, une émulsion ou encore sous la forme d'un surnageant. La matière première est préférentiellement choisie dans le groupe comprenant (constitué de) de cellules (d'origine humaine ou animale), de bactéries, d'agents infectieux, de virus, de (micro)organismes (vivants), de levures, d'algues, de prions, et de leurs mélanges. De préférence, un ingrédient ou excipient pouvant être utilisé dans la composition de solutions stériles peut aussi constituer la matière première. De manière préférée la matière première peut être sous forme solide et ensuite mise en solution avant introduction dans le dispositif selon l'invention ou cette mise en solution est réalisée lorsque le contenant est introduit dans le dispositif selon l'invention (par ajout d'une solution via un conduit du moyen de prélèvement). Toutes les caractéristiques de ce paragraphe peuvent être combinées entre elles.

Par « (**micro)organisme** » on entend l'ensemble des organismes invisibles à l'oeil nu. Ce terme comprend les bactéries, les protozoaires, les algues microscopiques, les champignons microscopiques. Cette définition comprend également les cellules eucaryotes végétales, animales ou humaines, issues de tissus, lignées cellulaires cancéreuses ou non-cancéreuses. Doit être également compris dans cette definition l'ensemble des agents infectieux, vivants ou inertes, tels que les virus ou les prions. Les catégories précitées pouvant être génétiquement modifiées.

Par « **étanche** » on entend l'aptitude à ne pas laisser passer les fluides (liquides, gaz), les poussières ou encore l'humidité.

L'expression « **espace confinable** » doit être comprise comme signifiant que l'espace peut être confiné, par rapport à l'environnement extérieur, en particulier extérieur à celui de la chambre de réception ou vis-à-vis d'autres compartiments environnants. Cette caractéristique peut être reliée à l'ouverture obturable. Ainsi, lorsque cette ouverture est fermée, l'espace confinable devient confiné et, de préférence aussi étanche. Il est donc possible d'avoir accès à l'espace confinable au moyen de l'ouverture obturable qui laisse une certaine latitude à l'utilisateur au moment des manipulations (manuelles ou automatiques).

Cet espace confinable est agencé pour être décontaminé, en particulier avant prélèvement ou encore avant introduction du premier contenant dans la chambre de réception.

L'expression « **premier contenant** » peut être interprétée dans le cadre de la présente invention comme « le contenant (de préservation) » qui contient la matière première à prélever. Ainsi, ce type de premier contenant peut être présent en grande quantité (en série). Une pluralité de premiers contenants peut donc faire l'objet de la présente invention au vu des modes de réalisation exposés. Ainsi, plusieurs contenants peuvent être présents dans la chambre de réception afin de faire fonctionner le dispositif en série ou plusieurs premiers contenants peuvent être ajoutés individuellement, l'un après l'autre, en série.

L'expression « **deuxième contenant** » se rapporte à un type de contenant d'amplification. En d'autres termes, ce type de contenant peut également être présent en grande quantité (en série). Celui-ci permet une amplification de la matière première, en particulier une reproduction cellulaire. Par exemple, un bioréacteur peut faire office de deuxième contenant et comprendre un milieu de culture.

Comme expliqué ci-avant, l'industrie pharmaceutique ou biotechnologique aura recours au dispositif de prélèvement selon l'invention pour prélever et, éventuellement transférer une matière première (2). Cette dernière pouvant proliférer dans un milieu de culture. Cette matière première (2) peut être en solution, ou en suspension ou encore sous la forme d'un surnageant ou émulsion. Celle-ci est ainsi prelevée du premier contenant (3) avec le moyen de prélèvement (1), et éventuellement transférée vers un deuxième contenant (8) afin de permettre une prolifération dans un milieu de culture. Ceci est communément appelé «reproduction cellulaire» ou «culture bactérienne». Parmi ces mises en culture, l'homme du métier connait aussi la mise en culture d'agents infectieux, de cellules souches mésenchymateuses humaines (hMSC), de cellules CAR-T (en anglais: « Chimeric Antigenic Receptor - T ») qui sont multipliées dans le dessein d'être administrées au patient. En outre, une mise en culture peut être utilisée dans la production de vaccins, dans le domaine de la thérapie génique.

Selon la présente invention, il est possible de manière préférée d'utiliser un fluide de décontamination choisi dans le groupe comprenant de l'eau, du peroxyde d'hydrogène, de l'acide péracétique, et de leurs mélange.

De manière avantageuse, le fluide de séchage peut être de l'air comprimé.

De manière préférée, le fluide de rinçage peut être de l'eau.

Ainsi, le dispositif selon l'invention se prête à une utilisation dans le cadre de la reproduction cellulaire ou bactérienne ou la culture d'agents infectieux pour ne citer que quelques exemples.

Selon un mode préféré de la présente invention, un premier contenant (3) comprenant au moins une cellule en solution (2) est introduit dans l'ouverture obturable (6). Le contenant est fermé. Ensuite, le moyen d'accueil (7) qui est positionné selon un axe médian de la chambre de réception (4) est orienté en direction de l'ouverture (6) et reçoit le premier contenant (3) qui est maintenu de manière stable dans le moyen d'accueil (7). L'ouverture (6) est fermée à l'aide d'un élément de fermeture, comme un bouchon. Ainsi, la chambre de réception (4) est étanche, lorsque l'ouverture (6) est refermée hermétiquement, ce qui forme un espace confinable (5) dans la chambre de réception (4). La chambre de réception (4) est ensuite décontaminée à l'aide d'un fluide. Lorsque la chambre de réception (4) est décontaminée, le premier contenant (3) est orienté avec le moyen d'accueil (7) en direction du moyen de prélèvement (1). Le moyen de prélèvement (1) est positionné à l'intérieur de l'espace confinable (5) jusqu'à atteindre le fond du premier contenant (3). Il est à noter que le moyen de prélèvement (1) se situe dans un milieu confiné avant prélèvement. Par un système de pompage, la solution (2) est prélevée et transférée vers un deuxième contenant (8) qui contient un milieu de culture.

De manière avantageuse, la matière prélévée en solution est transférée de manière aseptique vers un deuxième contenant (8) qui présente un volume supérieur à celui du premier contenant (3). Ces étapes peuvent être réalisées plusieurs fois, c'est-à-dire en introduisant plusieurs contenants du même type que le premier contenant (3) en série dans la chambre de réception (4).

Il est à noter que le premier contenant (3) est prévu pour comprendre une matière première (2) agencée pour proliférer dans un milieu de culture. Le deuxième contenant (8) est lui prévu pour contenir du milieu de culture et la matière première (2) contenue dans le premier contenant (3).

Le volume du deuxième contenant (8) en cas de reproduction cellulaire augmentera préférentiellement selon un processus d'amplification complet (appelée communément procédé «end to end») de façon à augmenter la quantité de matière amplifiée jusqu'au volume final souhaité. Le but étant de fournir avantageusement et en finalité une solution injectable, de préférence dans le domaine pharmaceutique ou pour des applications biotechnologiques.

Selon un mode préféré, le dispositif selon l'invention est donc agencé pour permettre de produire de manière aseptique une solution injectable dans le domaine pharmaceutique ou pour des applications biotechnologiques.

De préférence, le dispositif selon l'invention permet une fabrication de médicament, d'excipient, de formulation galénique ou encore une dilution d'une matière première.

De manière alternative, il est également possible d'avoir une chambre de réception (4) qui présente plusieurs moyens d'accueil (7) de façon à pouvoir prélever de la matière première (2) à partir de plusieurs contenants (3).

Le dispositif de prélèvement de la présente invention permet un prélèvement et éventuellement un transfert d'une matière première (2) qui est agencée pour proliférer dans un milieu de culture de manière aseptique. Le prélèvement aseptique permet d'éviter toute contamination du contenu du premier contenant par des contaminants non désirés, tels que des germes du type microorganisme. Cela a pour avantage de pouvoir augmenter les rendements de productions, grâce à la limitation de gaspillage de matière première (2) résultant d'éventuelles contaminations.

Le dispositif selon la présente invention comprend une chambre de réception (4) étanche lorsque l'ouverture obturable (6) est refermée. L'étanchéité permet de ne pas laisser passer les fluides (liquides ou gaz), les poussières, l'humidité. Cela a ainsi pour but de créer un espace confiné (5) par rapport au milieu environnant, extérieur à la chambre de réception (4), et éventuellement extérieur au moyen de prélèvement (1).

Il est à noter que l'utilisateur peut être remplacé par un système automatique, robotisé ce qui permet de s'affranchir de la présence de celui-ci.

Aussi, ledit moyen d'accueil (7) est orientable dans l'espace confinable (5), ce qui permet de l'orienter à souhait dans l'espace confinable (5). Cela permet ainsi avantageusement de positionner le moyen d'accueuil (7) face à l'ouverture obturable (6), d'orienter ce premier contenant (3) en direction du moyen de prélèvement (1), ou encore de manière préférée de permettre d'orienter ce premier contenant (3) dans un emplacement de la chambre de réception (4) pour éventuellement briser une des extérmités d'un contenant (en verre) afin d'avoir accès à son contenu.

De manière avantageuse, le dispositif selon l'invention est stérilisé par gamma irradiation, de préférence avant toute manipulation de prélèvement, ou encore avant toute introduction du premier contenant (3) dans le dispositif.

Le dipositif selon l'invention permet une amplification ou prolifération de la matière première (2). De manière préférée, il est aussi possible d'utiliser le dispositif selon l'invention pour diluer la matière première (2) ou encore pour faire de la formulation ou des tests de stérilité.

D'autres caractéristiques, détails et avantages de l'invention ressortiront de la description donnée ci-après, à titre non limitatif et en faisant référence aux dessins et aux exemples.

Les processus de type « end-to-end » peuvent aussi intégrer le dispositif selon l'invention et consistent à intégrer toutes les étapes d'un process de production depuis la matière première jusqu'au produit fini. Ainsi, le dispositif selon l'invention permet d'intégrer la première étape d'un processus appelé « end-to-end ».

Les références de chaque élément repris dans la présente invention sont les suivants :
Moyen de prélèvement (1)
Extrémité du moyen de prélèvement (1a)
Matière première à prélever (2)
Premier contenant (3)
Extrémité proximale du premier contenant (3a)
Chambre de réception (4)
Espace confinable (5)
Ouverture obturable (6)
Moyen d'accueil (7)
Première partie du moyen d'accueil (7a)
Deuxième partie du moyen d'accueil (7b)
Extrémité distale du moyen d'accueil (7c)
Deuxième contenant (8)
Element refermable (9) - assurant l'étanchéité
Moyen de guidage (10) - deuxième partie du moyen d'accueil (7b)
Chambre de prélèvement (11)
Partie flexible (11a) de la chambre de prélèvement (11)
Moyen de guidage (12) du moyen de prélèvement (1)
Gorge (12a) du moyen de guidage (12)
Surface extérieure (12b) du moyen de guidage (12)
Corps (13) du moyen de prélèvement (1)
Aiguille (14) du moyen de prélèvement (1)
Partie flexible (15) de la chambre de réception (4)
Moyen d'alimentation d'au moins un fluide de décontamination (16)
Connexion fluidique d'au moins un fluide de décontamination (16a)
Moyen d'alimentation d'au moins un fluide de rinçage (17, 27)
Connexion fluidique d'au moins un fluide de rinçage (17a)
Premier moyen d'évacuation de fluide (18)
Moyen d'alimentation d'un fluide de séchage muni d'une membrane filtrante et d'un filtre hydrophobe (19)
Connexion fluidique d'au moins un fluide de séchage (19a)
Connexion fluidique de décontamination de la connexion fluidique d'au moins un fluide de séchage (19b)
Deuxième moyen d'évacuation de fluide (20)
Partie rigide 21 (rigidité définie par rapport à la partie flexible (15)) de la chambre de réception (4)
Moyen d'ouverture (22) du premier contenant (3)
Moyen de réception d'au moins un fluide de purge de filtre (24) Connexion fluidique d'au moins un fluide de purge de filtre (24a) Moyen de stockage des fluides usagés (25)
Moyen de filtration (26) avec un filtre hydrophile
Moyen d'alimentation d'au moins un fluide de rinçage (27) pour le moyen de prélèvement (1)
Connexion fluidique d'au moins un fluide de rinçage (27a) pour le moyen de prélèvement (1)
Conduit de prélèvement (27b, 27c)
Moyen d'éventage du contenant (8) et d'aspiration de la matière première (2)
Moyen de pompage, pompes (A)
Filtre hydrophobe ou hydrophile (F) en fonction de l'utilisation visée

La figure 1 est une vue schématique d'un mode de réalisation du dispositif selon la présente invention.
La figure 2 est une vue schématique d'un mode de réalisation préféré du dispositif selon la présente invention.
Les figures 3 à 10 sont des illustrations du dispositif de la figure 2 selon différentes étapes de manipulations.

Sur les figures, les éléments identiques ou analogues portent les mêmes références.

D'autres caractéristiques et avantages de la présente invention seront tirés de la description non limitative qui suit, et en faisant référence aux dessins et aux exemples.

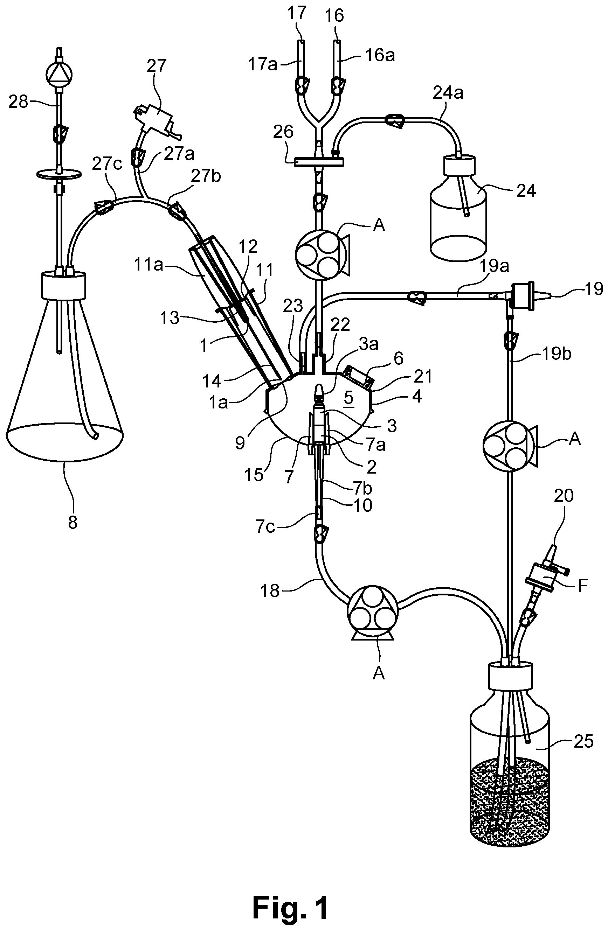

Le figure 1 illustre un dispositif de prélèvement aseptique selon un mode de réalisation de l'invention qui est agencé pour permettre le prélèvement d'une matière première (2). Cette matière première (2) peut être une cellule ou un (micro)organisme en suspension.

Le dispositif de la figure 1 comprend **un moyen de prélèvement** (1) d'une matière première en solution et **une chambre de réception** (4) étanche qui présente un espace confiné (5). Le moyen de prélèvement (1) est adjacent à la chambre de réception (4) qui est munie d'une **ouverture (6)** obturée par un bouchon et d'un **moyen d'accueil** (7). De plus, il apparaît que le moyen de prélèvement (1) est séparé de la chambre de réception (4) par un élément refermable, en l'espèce par un septum (9). Aussi, le moyen de prélèvement (1) se situe dans une **chambre de prélèvement** (11) qui présente un moyen de guidage (12). Ce moyen de guidage (12) se présente sous la forme d'un cône qui est relié au moyen de prélèvement (1) au niveau du corps (13), par exemple un embout (14), qui est muni à l'une de ses extrémités d'une aiguille (14) et, éventuellement à l'autre extrémité d'un corps de pompe (ou d'un conduit).

Le moyen de prélèvement (1) présente ainsi un corps (13) étanche muni à l'une de ses extrémités d'une aiguille (ou tout autre moyen pouvant laisser passer la matière première présente dans le premier contenant (3)). Le corps (13) est également muni à l'autre extrémité, opposée à l'aiguille, d'un conduit (27b). Ainsi, le corps (13), l'aiguille (14) et le conduit (27b) sont en communication fluidique.

La chambre de prélèvement (11) est confinée et étanche, ce qui est donc aussi le cas du moyen de prélèvement (1) qui se trouve dans un environnement confiné et séparé de l'espace confiné (5) de la chambre de réception (4).

Aussi, la chambre de prélèvement (11) comprend un corps flexible (11a), qui fonctionne de préférence comme un accordéon, qui présente une position de repos (forme tendue d'un accordéon) et une position de prélèvement (forme contractée d'un accordéon). Ainsi, lorsque le corps flexible (11a) est en position de repos, tout le moyen de prélèvement (1) se situe dans la chambre de prélèvement (11) comme illustré à la figure 1. Lorsque le corps flexible (11a) est en position de prélèvement, celui-ci est dans une position où il est replié sur lui-même (comme un accordéon en position contractée) et le moyen de prélèvement (1) se situe alors dans l'espace confiné (5) de la chambre de réception (4). Quand le prélèvement est réalisé, le corps flexible (11a) est agencé pour passer à nouveau en position de repos (avec l'accordéon en position tendue).

De manière alternative, ce corps flexible (11a) peut aussi être un piston qui permet aussi de fournir un environnement confiné dans lequel se situe le moyen de prélèvement. Le fonctionnement indiqué ci-avant est alors similaire s'agissant des positions de repos et de prélèvement.

La chambre de réception (4) présente au moins une partie de sa surface en matière flexible (15), ladite partie étant éventuellement adjacente audit moyen d'accueil (7), qui traverse cette partie (15) de manière étanche, de préférence selon un axe médian de la chambre de réception (4). La figure 1 représente une chambre de réception (4) qui présente une partie de sa surface en matière flexible (15) et une autre partie en matière rigide (21) (cette rigidité étant définie par rapport à la matière flexible). Ainsi, la surface en matière flexible (15) peut être faite en un plastique malléable et la matière plus rigide (15) en élastomère, de préférence en caoutchouc (moins malléable).

Le moyen d'accueil (7) de la chambre de réception (4) comprend une première partie (7a) situé dans l'espace confinable (5) qui maintient (sans intervention manuelle) de manière stable le premier contenant (3) qui contient une matière première (2) à prélever, par exemple des cellules en suspension (2). Le premier contenant (3) présente une partie proximale 3a (en verre) et est fermé lorsqu'il est dans l'espace confiné (5). Cette première partie (7a) est reliée à une deuxième partie (7b) qui, elle, est située à l'extérieur de la chambre de réception (4). Cette deuxième partie (7b) s'étend à l'extérieur de ladite chambre de réception (4) sous la forme d'un moyen de guidage (10), pouvant être orienté par rapport à un axe médian qui traverse ladite chambre de réception (4).

De plus, la deuxième partie (7b) du moyen d'accueil (7) présente une extrémité distale (7c) reliée à un premier moyen d'évacuation d'au moins un fluide (18). Ainsi, le moyen d'accueil (7) est en communication fluidique avec l'espace confiné (5) de la chambre de réception (4).

De manière alternative, l'extrémité distale (7c) est obturée, sans être reliée au premier moyen d'évacuation d'au moins un fluide (18). Dans ce mode de réalisation, le moyen d'évacuation d'au moins un fluide (18) se situe ailleurs sur la chambre de réception (4), de façon à être séparé de l'extrémité distale (7c) du moyen d'accueil (7).

Aussi, le moyen d'accueil (7) se situe sur la partie de la chambre de réception (4) qui est en matière flexible (15).

La chambre de réception (4) comprend en outre, et de préférence sur sa partie en matière rigide, l'ouverture obturée (6), un moyen d'ouverture (22) du premier contenant (3) et au moins une connexion fluidique (23) d'un fluide.

Le moyen d'ouverture (22) est apte à accueillir la partie proximale (3a) du premier contenant (3) et est agencé pour briser la partie proximale (3a) du premier contenant (3) par un mouvement de pivotement.

En fonction du type de moyen de fermeture du premier contenant (3), le moyen d'ouverture (22) aura une forme et une fonction qui permettra l'accès au premier contenant (3) de manière aseptique. Ce type de moyen de fermeture peut être un bouchon, une membrane, une capsule, etc.

Il est évident, que lorsque le premier contenant (3) est muni d'un septum à l'extrémité proximale (3a) du premier contenant (3), le moyen d'ouverture (22) n'est pas utilisé (voire présent) dans la chambre de réception (4).

Comme illustré à la figure 1, le moyen d'ouverture (22) peut aussi être relié à une connexion fluidique qui est reliée à un moyen de filtration (26), ici un filtre hydrophile (26). Ce moyen de filtration (26) permet de filtrer les fluides, ici les fluides de rinçage et/ou de décontamination pour limiter les contaminations et l'encrassement de la chambre de réception (4). Ce moyen de filtration (26) est relié à une connexion fluidique d'au moins un fluide de purge de filtre (24a) qui se présente sous la forme d'un conduit (24a) qui atterit dans un contenant (24) faisant office de moyen de réception d'au moins un fluide de purge de filtre (24).

Le moyen de filtration (26) est aussi connecté à un moyen d'alimentation d'un fluide de décontamination (peroxide d'hydrogène) (16) via une connexion fluidique de décontamination (16a). De plus, le moyen de filtration (26) est relié à un moyen d'alimentation de fluide de rinçage (eau) (17) via une connexion fluidique de rinçage (17a).

De manière alternative, lorsque le moyen d'ouverture (22) n'est pas présent, les connexions d'alimentation (16,17), le moyen de filtration (26) et leurs conduits respectifs (16a, 17a, 24a) sont agencés pour être en communication fluidique avec la chambre de réception (4).

Dans le mode de réalisation illustré à la figure 1, la chambre de réception (4) est également connectée fluidiquement à un moyen d'alimentation en fluide de séchage (air) (19). Ainsi l'embout (23) de la chambre de réception (4) est relié par un conduit (19a) au moyen d'alimentation en fluide de séchage (air) (19). Ensuite, le moyen d'alimentation en fluide de séchage (19) est également connecté à un conduit (19b) qui permet par l'intermédiaire d'une pompe (A) d'évacuer les fluides de la chambre de réception (4) suite à un séchage vers un contenant de réception (par mise au rebut, contenant (25)).

Le dispositif illustré comprend également un moyen d'évacuation de fluide (ici d'air) (20) qui peut par exemple se situer dans le contenant (25) ou ailleurs en fonction de l'agencement de l'ensemble des éléments formant le dispositif selon l'invention.

De manière additionnelle, le corps (13), l'aiguille (14) et le conduit (27b) sont en communication fluidique. Le conduit de prélèvement (27b, 27c) permet de transférer la matière première (2) en solution (cellule en suspension) vers un deuxième contenant (8). Il est aussi possible de rincer le moyen de prélèvement (1) grâce à un moyen d'alimentation d'un fluide de rinçage (27). Le dispositif peut aussi inclure un moyen d'évacuation (28), ou d'éventage (28).

En pratique, le deuxième contenant peut contenir un milieu de culture (non illustré).

Le deuxième contenant (8), adjacent à ladite chambre de réception (4), est relié par ledit moyen de prélèvement (1) à ladite chambre de réception (4), par lequel la matière première (2) d'intérêt est transférée.

Le deuxième contenant (8) présente un volume supérieur à celui du premier contenant (3) et est agencé pour permettre une mise en culture, lorsque ladite matière première (2) prélevée dudit premier contenant (3) est transférée dans ledit deuxième contenant (8).

La figure 1 comprend ainsi plusieurs conduits prévus pour laisser passer au moins un fluide (16a, 17a, 18, 19a, 19b, 24a, 27a, 27b, 27c, 28), plusieurs pompes (A), ainsi que des éléments et filtres hydrophiles ou hydrophobes (19, 20, 24, 25, 26, 27, F) communément utilisés dans le domaine technique de la présente invention et qui peuvent être relié au dispositif de l'invention de plusieurs façon.

En fonctionnement, un liquide de décontamination (peroxyde d'hydrogène) est alimenté par le moyen d'alimentation de fluide (16), jusqu'à remplir partiellement la chambre de réception (4).

Ensuite, le bouchon de l'ouverture (6) est retiré et un flacon aseptique (3) (fourni par la société Aseptique Technologies) contenant des cellules en suspension (2) est introduit dans la chambre de réception (4), dans laquelle le moyen d'accueil (7) est orienté au niveau de sa deuxième partie (7b) en direction de l'ouverture (6). Ainsi, le flacon (3) est logé dans le moyen d'accueil (7) de manière stable. Lorsque le moyen d'accueil (7) est orienté, la surface flexible (15) de la chambre de réception (4) se déforme pour permettre une orientation adéquate. Lorsque le flacon (3) est maintenu dans le moyen d'accueil (7), ce dernier se positionne, de préférence verticalement selon un axe médian de la chambre de réception (4).

Une étape de décontamination est alors réalisée en alimentant la chambre de réception (4) en fluide de décontamination (peroxyde d'hydrogène) jusqu'à complètement la remplir de fluide de décontamination. Dès que la décontamination est terminée, une vidange est réalisée afin d'éliminer le fluide de décontamination (conduit (18) dans le contenant de mise au rebut (25).

Après l'étape de décontamination, un remplissage complet de la chambre de réception (4) est réalisé avec un fluide de rinçage (eau) à l'aide du moyen d'alimentation de fluide de rinçage (17) afin d'éliminer toute trace de fluide de décontamination. S'ensuit alors une étape de vidange vers le contenant de mise au rebut (25). De préférence, cette étape de rinçage est dynamique en ce qu'elle élimine le liquide de décontamination et ne se limite donc pas à la diluer.

Une étape de séchage a alors lieu par le moyen d'alimentation en fluide de séchage (19), de manière préférée par introduction d'air comprimé.

Lorsque le flacon (3) doit être brisé à son extrémité proximale (3a), celui-ci est orienté dans le moyen d'ouverture (22) qui va permettre à la suite d'un mouvement de rotation / pivotement de briser cette extrémité afin de permettre un accès aux cellules en suspension (2) à prélever. Cette étape est facultative et pas nécessaire lorsque l'accès à la matière première (2) à prélever ne nécessite pas de briser une partie du contenant (3). Par exemple, lorsque le contenant (3) est muni d'un septum ou de tout autre élément refermable qui permet l'accès au contenu du flacon sans cassure.

Ensuite, le moyen d'accueil (7) logeant le flacon ouvert est orienté en direction du moyen de prélèvement (1), de préférence selon un axe médian de la chambre de prélèvement (11). Ainsi, l'ouverture du flacon se situe en face du septum (9).

Comme expliqué ci-avant, le corps flexible (11a) de la chambre de prélèvement va permettre d'introduire l'aiguille (14) du moyen de prélèvement (1) dans l'espace confiné (5) de la chambre de réception (4) en passant d'une position de repos (forme tendue d'un accordéon, comme illustré à la figure 1) vers une position de prélèvement (forme contractée d'un accordéon). Lorsque le corps flexible (11a) est en position de prélèvement, celui-ci est dans une position où il est replié sur lui-même (comme un accordéon en position contractée) et le moyen de prélèvement (1) se situe alors dans l'espace confiné (5) de la chambre de réception (4), plus exactement au fond du flacon (3) prêt à prélever les cellules en suspension.

Par un système de pompage ou d'aspiration ou de piston ou tout autre système de prélèvement qui garantit un prélèvement aseptique, les cellules en suspension sont ainsi prélevées et transférées vers le deuxième contenant (8). Ce dernier comprend un milieu de culture qui va être mélangé avec les cellules en suspension de façon à permettre une reproduction cellulaire. Quand le prélèvement est réalisé, le corps flexible (11a) est agencé pour passer à nouveau en position de repos (avec l'accordéon en position tendue).

Ainsi, l'aiguile (14) peut être remplacée par un conduit qui permettrait un prélèvement et transfert efficace. En effet, l'aiguille a un rôle plutôt passif dans le dispositif étant donné qu'elle n'assure pas le retrait des cellules en suspension mais permet plutôt un accès aisé et efficace jusqu'au fond du flacon. Tout autre moyen équivalent conviendrait également.

Ce type de manipulation peut ainsi être utilisé pour permettre de manière générale dans un système fermé (fournit par le dispositif selon l'invention) d'un moyen de prélèvement aseptique.

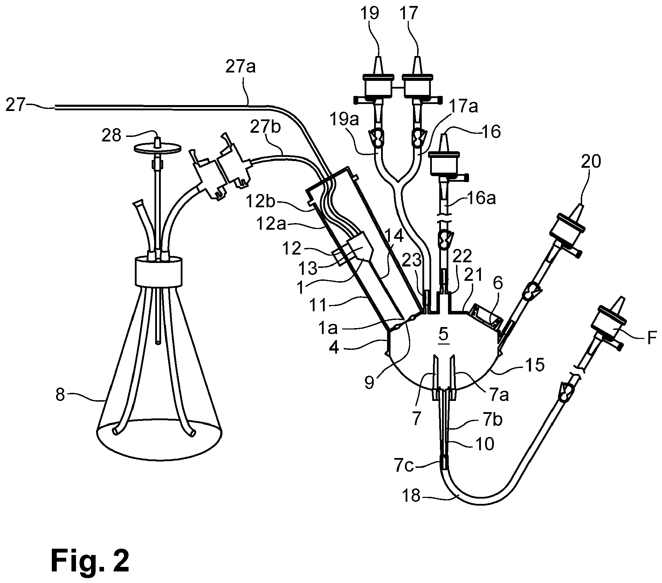

La figure 2 est similaire à la figure 1 et illustre un autre mode de réalisation du dispositif selon l'invention en reprenant une partie des références de la figure 1, excepté que le moyen de prélèvement (1) est différent. Le moyen de prélèvement 1 se situe dans une chambre de prélèvement (11) qui présente un moyen de guidage (12) qui permet de déplacer ledit moyen de prélèvement (1), de préférence selon un axe médian de ladite chambre de prélèvement (11) en direction de ladite chambre de réception (4). Le moyen de guidage (12) présente une gorge (12a) qui s'étend longitudinalement sur une surface extérieure (12b) dudit moyen de guidage (12) pour faire coulisser ledit moyen de prélèvement (1) pour qu'une des extrémités (1a) dudit moyen de prélèvement (1) soit introduite dans ladite chambre de réception (4). En l'espèce, l'aiguille peut ainsi être introduite dans l'espace confinable (5) de la chambre de réception (4).

Comme illustré, le corps (13) est muni d'un moyen de guidage (12), en forme de saillie, qui s'étend jusqu'en dehors de la chambre de prélèvement (11).

Dans ce mode de réalisation, un corps flexible (11a) n'est pas nécessaire, ce qui permet d'avoir une chambre de prélèvement (11) en partie rigide.

Le fonctionnement du dispositif illustré à la figure 1 est décrit ci-avant est également applicable ici.

Il est à noter que concernant les différentes étapes de manipulations liées à la décontamination, rinçage et séchage. L'homme du métier pourra combiner ces étapes comme il le souhaite en fonction des besoins lors de la manipulation.

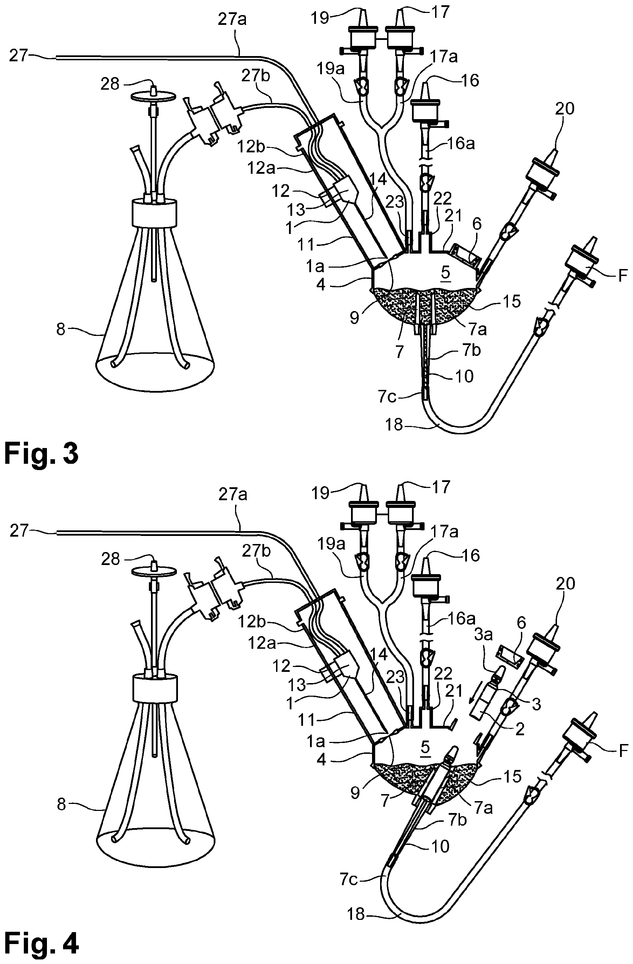

Les figures 3-10 illustrent la façon dont le dispositif peut être utilisé, en particulier lorsqu'un rinçage et/ou ou une décontamination et/ou séchage a lieu.

Ces figures 2-10 peuvent également être comprise comme illustrant de manière séquentielle la façon dont le dispositif est mis en œuvre.

La figure 3 illustre le dispositif selon un mode préféré lorsque un fluide de décontamination (comme du peroxyde d'hydrogène) est introduit dans la chambre de réception (4) qui est partiellement remplie de liquide de décontamination. Ce fluide pouvant aussi être un fluide de rinçage, comme de l'eau.

La figure 4 illustre le dispositif selon un mode avantageux, dans lequel une étape de positionnement du premier contenant (3) dans la chambre de réception (4) est réalisée par ledit moyen d'accueil (7) qui est orienté vers l'ouverture obturable (6).

Selon un mode particulièrement avantageux (non illustré), dans lequel le premier contenant (3) est positionné selon un axe médian de la chambre de réception (4), en position verticale, lorsqu'un fluide de décontamination (comme du péroxide d'hydrogène) rempli partiellement la chambre de réception (4) ou totalement. Ce fluide peut être un fluide de rinçage (comme de l'eau). Cette étape pouvant se situer à la suite de celle illustrée à la figure 4.

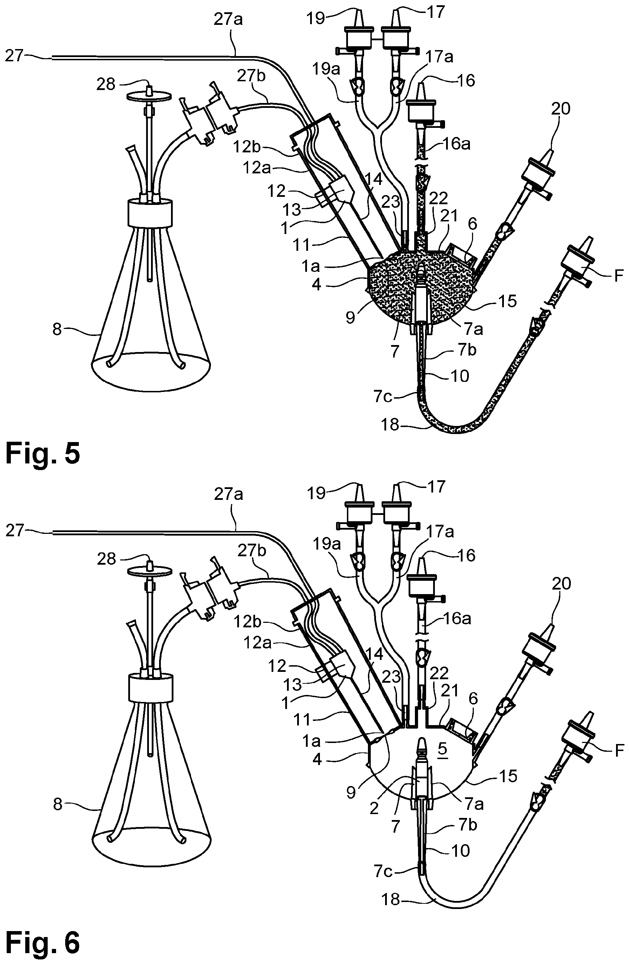

A la figure 5 est illustrée une étape de décontamination complète par remplissage de toute la chambre de réception (4) par un fluide de décontamination.

La figure 6 illustre un mode de réalisation avantageux, dans lequel est réalisée une étape de vidange du fluide de décontamination (ou de rinçage) de la chambre de réception (4).

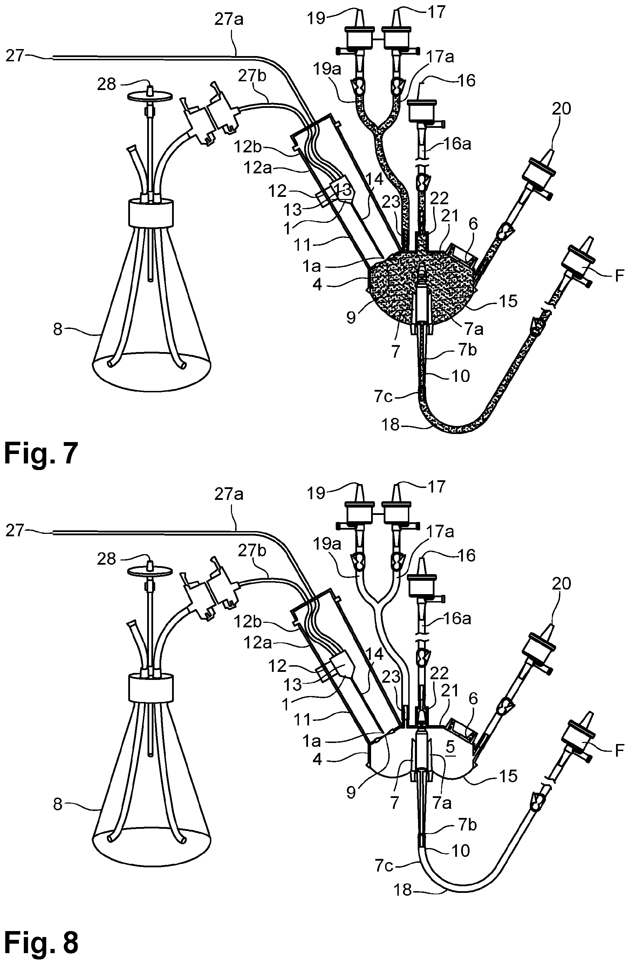

A la figure 7 est illustrée une étape de rinçage complet par remplissage de toute la chambre de réception (4) par un fluide de rinçage.

La figure 8 illustre un mode dans lequel est illustré la flexibilité d'une partie de la chambre de réception (4). Est réalisé une étape d'ouverture du premier contenant (3) après positionnement du premier contenant (3) dans le moyen d'ouverture (22).

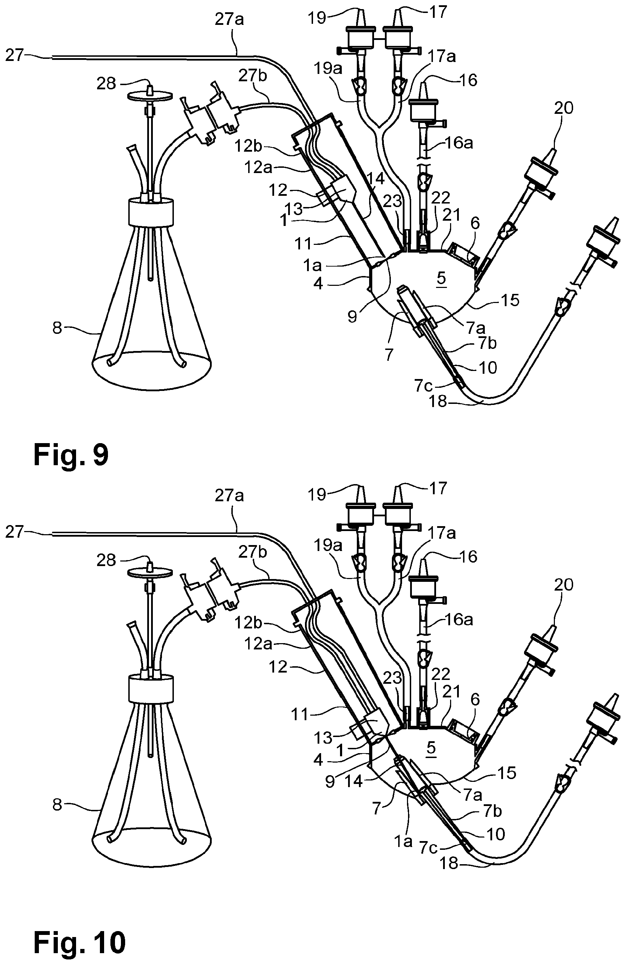

La figure 9 illustre un mode particulier, dans lequel est réalisé une étape de positionnement de l'ouverture du premier contenant (3) en face du moyen de prélèvement (1) par pivotement du moyen d'accueil (7).

La figure 10 illustre une réalisation préférée dans laquelle le moyen de prélèvement (1) est placé à l'intérieur du premier contenant (3) pour permettre un prélèvement.

Le mode de réalisation illustré à la figure 8 n'est pas essentielle lorsque le premier contenant (3) est muni d'un élément refermable, comme un septum ou tout élément équivalent.

Sur les figures, les éléments identiques ou analogues portent les mêmes références.

Dans le cadre de la présente invention, tout article singulier comme par exemple , « un », « une », « le », « la », « de », « du » peut être remplacé par un article qui désigne un pluriel comme par exemple au « moins 2 », « au moins 3 », « plusieurs » etc.

Le mot « comprendre », « contient » ou tout terme équivalent ou dérivés peut être remplacé par « constitué de » afin de définir une liste ou des possibilité de sélection exclusive afin de ne pas englober d'autres éléments non-cités dans l'expression utilisée.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Dispositif de prélèvement aseptique pour une prolifération d'une matière première dans un milieu de culture ou pour une mise en culture cellulaire ou microbienne, le dispositif comprenant :
- Un moyen de prélèvement (1) d'une matière première (2), de préférence en solution, agencée pour proliférer dans un milieu de culture, ledit moyen de prélèvement (1) étant agencé pour prélever de manière aseptique la matière première (2) qui est contenue dans un premier contenant (3) d'un volume prédéterminé, et
- Une chambre de réception (4) qui présente un espace confinable (5) et qui est agencée pour permettre une décontamination de celle-ci par le passage d'au moins un fluide dans ledit espace confinable (5), ladite chambre de réception (4) étant munie :
∘ d'une ouverture obturable (6) agencée pour un passage dudit premier contenant (3) dans ledit espace confinable (5) et, lorsqu'elle est obturée, est agencée pour mettre en confinement la chambre de réception (4),
∘ d'un moyen d'accueil (7) orientable dans l'espace confinable (5) et étant agencé pour réceptionner ledit premier contenant (3) et le positionner en direction dudit moyen de prélèvement (1), ce dernier étant agencé pour prélever de manière aseptique ladite solution (2) contenue dans ledit premier contenant (3) et agencé pour la transférer de manière aseptique vers un deuxième contenant (8) présentant un volume supérieur au volume dudit premier contenant (3).

2. Dispositif selon la revendication 1, dans lequel ledit moyen de prélèvement (1) est adjacent audit espace confinable (5) de ladite chambre de réception (4) avant prélèvement, et est de préférence dans un milieu confiné et étanche.

3. Dispositif selon l'une quelconque des revendications précédentes, dans lequel ledit moyen de prélèvement (1) est séparé de ladite chambre de réception (4) par un élément refermable (9) choisi dans le groupe comprenant un septum, une membrane, et une électrovanne.

4. Dispositif l'une quelconque des revendications précédentes, dans lequel, lors dudit prélèvement, une partie dudit moyen de prélèvement (1) se situe dans l'espace confinable (5) de la chambre de réception (4).

5. Dispositif selon l'une quelconque des revendications précédentes, dans lequel ledit moyen de prélèvement (1) se situe dans une chambre de prélèvement (11) qui présente un moyen de guidage (12) dudit moyen de prélèvement (1).

6. Dispositif selon la revendication 5, dans lequel ledit moyen de guidage (12) est agencé pour déplacer ledit moyen de prélèvement (1) selon un axe médian de ladite chambre de prélèvement (11) en direction de ladite chambre de réception (4).

7. Dispositif selon la revendication 5 ou 6, dans lequel ledit moyen de guidage (12) présente une gorge (12a) qui s'étend longitudinalement sur une surface extérieure (12b) dudit moyen de guidage (12) pour faire coulisser ledit moyen de prélèvement (1) pour qu'une des extrémités (1a) dudit moyen de prélèvement (1) soit introduite dans ladite chambre de réception (4).

8. Dispositif selon l'une quelconque des revendications précédentes, dans lequel ledit moyen de prélèvement (1) présente un corps (13) agencé pour permettre le passage de ladite matière première (2) et qui est muni à l'une de ses extrémités d'un moyen d'aspiration (14), de préférence d'une aiguille (14) ou d'un conduit.

9. Dispositif selon l'une quelconque des revendications précédentes, dans lequel ladite chambre de prélèvement (11) est confinée et étanche.

10. Dispositif selon l'une quelconque des revendications précédentes, dans lequel ladite chambre de réception (4) présente au moins une partie de sa surface en matière flexible (15), ladite partie étant éventuellement adjacente audit moyen d'accueil (7), qui est agencé pour traverser ladite au moins une partie (15) de la surface de la chambre de réception (4) en matière flexible.

11. Dispositif selon l'une quelconque des revendications précédentes, comprenant un deuxième contenant (8) adjacent à ladite chambre de réception (4) et qui est reliée par ledit moyen de prélèvement (1) à ladite chambre de réception (4).

12. Dispositif selon la revendication 11, dans lequel ledit deuxième contenant (8) présente un volume supérieur à celui du premier contenant (3) et étant agencé pour permettre une mise en culture, lorsque ladite matière première (2) prélevée dudit premier contenant (3) est transférée dans ledit deuxième contenant (8).

13. Dispositif selon l'une quelconque des revendications précédentes, dans lequel ledit moyen d'accueil (7) présente une première partie (7a) qui s'étend dans ledit espace confinable (5) et une deuxième partie (7b) qui s'étend à l'extérieur de ladite chambre de réception (4) sous la forme d'un moyen de guidage (10), de préférence selon un axe médian qui traverse ladite chambre de réception (4).

14. Dispositif selon l'une quelconque des revendications précédentes, dans lequel ladite partie sous forme de moyen de guidage (10) est en communication fluidique avec ladite chambre de réception (4).

15. Dispositif selon l'une quelconque des revendications précédentes, dans lequel ladite chambre de réception (4) est agencée pour être en communication fluidique avec au moins un des éléments suivants ou pris en combinaison:
- Un moyen d'alimentation d'au moins un fluide de décontamination (16)
- Un moyen d'alimentation d'au moins un fluide de rinçage, (17)
- Un moyen d'alimentation d'au moins un fluide de séchage (19),
- Un premier moyen d'évacuation d'au moins un fluide(18), ledit moyen d'évacuation (18) étant éventuellement directement relié à l'une des extrémités (7c) dudit moyen d'accueil (7),
- Et un deuxième moyen d'évacuation de fluide de séchage (20).
